# EUROPÄISCHE PATENTSCHRIFT

(11) **EP 0 779 226 B1**
(45) Veröffentlichungstag und Bekanntmachung des Hinweises auf die Patenterteilung: **25.08.1999**
(21) Anmeldenummer: 96120165.4
(22) Anmeldetag: 16.12.1996
(51) Int. Cl.: B65D 43/16, B65D 83/02

(54) **Vorratsbehältnis für streifenförmige Testelemente**
Storage container for strip-shaped test elements
Récipient de stockage pour éléments de test en forme de bande

(30) Priorität: 14.12.1995 DE 19546684
(43) Veröffentlichungstag der Anmeldung: 18.06.1997
(73) Patentinhaber: Roche Diagnostics GmbH, 68298 Mannheim (DE)
(72) Erfinder: Sacherer, Klaus-Dieter, 67281 Kirchheim (DE); Bainczyk, Gregor, Dr., 68199 Mannheim (DE)

(56) Entgegenhaltungen:
- EP-A- 0 538 067
- DE-A- 3 411 920
- DE-A- 4 328 815
- US-A- 3 701 452
- US-A- 4 717 018

## Beschreibung

Die Erfindung betrifft Vorratsbehältnisse für streifenförmige Testelemente enthaltend
- einen Köcher, der eine Öffnung besitzt, die durch einen Rand begrenzt ist,
- Testelemente, die sich im Köcher befinden,
- ein Verschlußteil, das über ein Scharnier mit dem Köcher verbunden ist und das an seiner Innenseite eine elastische Dichtung besitzt, die bei geschlossenem Vorratsbehältnis deformiert ist und dichtend an dem Rand des Köchers anliegt und die bei geöffnetem Vorratsbehältnis nicht deformiert ist, und
- eine lösbare Haltevorrichtung, die das Vorratsbehältnis in der geschlossenen Position hält.

Im Stand der Technik sind für die Bevorratung von Testelementen eine Reihe von Behältnissen bekannt. Trotz einer relativ schlechten Handhabbarkeit wird ein Vorratsbehältnis in Form einer Röhre mit einem Dichtungsstopfen, der an seiner Innenseite ein Trockenmittel besitzt, noch vielfach eingesetzt. Während diese Verpackungsform die Teststreifen sehr effektiv vor Feuchtigkeit, mechanischer Beanspruchung und Verschmutzung schützt, besitzt sie den Nachteil, daß der Verschlußstopfen recht schwergängig ist, wenn er genügend dichten soll. Weiterhin ist die Entnahme von Teststreifen aus der geöffneten Röhre ein Prozeß, der insbesondere unterzuckerten Diabetikern Schwierigkeiten bereitet. Der größte Teil der im Handel vertriebenen Teststreifen wird jedoch von diesem Personenkreis verwendet.

Aus EP-A 0 538 067 ist ein Behältnis für Ton- oder Videobandcassetten bekannt, welches in einem sogenannten Hart-/Weich-Spritzgußverfahren hergestellt ist. Das beschriebene Behältnis besitzt einen im wesentlichen quaderförmigen Köcher, der zur Aufnahme einer Cassette dient, und eine Klappe, die über ein Scharnier mit dem Köcher verbunden ist und zum Verschließen des Behältnisses dient. Die Klappe besitzt an ihrer Innenseite eine elastische Dichtung, die bei geschlossenem Behältnis dichtend am Köcher anliegt und so das Eindringen von Staub und Feuchtigkeit in das Behältnisinnere verhindert. Mittels einer lösbaren Haltevorrichtung können Klappe und Köcher geschlossen gehalten werden.

In der US-Patentanmeldung US-4,717,018 wird ein Vorratsbehältnis für Testelemente beschrieben, bei dem sich ein Eindrückstopfen zum Verschluß der Gefäßöffnung direkt am Gefäß selbst befindet, so daß der Stopfen nicht verlorengehen kann. Auch bei dieser Ausführungsform ist ein relativ großer Kraftaufwand zum Öffnen und Schließen des Verschlußstopfens notwendig. Weiterhin erfolgt die Entnahme von Teststreifen, indem das Behältnis gekippt wird und die Teststreifen durch die Öffnung rutschen. Ein Benutzer dieses Vorratsbehältnisses muß dafür Sorge tragen, daß die Teststreifen beim Herauskippen nicht herunterfallen und verschmutzt werden, was ihre bestimmungsgemäße Verwendung als diagnostisches Testmittel unmöglich machen könnte.

In der deutschen Offenlegungsschrift DE 43 28 815 ist ein System zur Bevorratung von Testelementen beschrieben, bei dem die Öffnung des Behältnisses über einen Schieber erfolgt, mit dem der Gefäßdeckel weggeschoben wird. Dieses System ist einfach und mit geringem Kraftaufwand zu bedienen. Das Vorratsbehältnis besitzt jedoch eine verhältnismäßig aufwendige Mechanik, die entsprechende Fertigungskosten nach sich zieht. Daher ist dieses System insbesondere als Nachfüllverpackung konzipiert.

Aufgabe der vorliegenden Erfindung war es, ein Vorratsbehältnis für streifenförmige Testelemente zur Verfügung zu stellen, das einfach und mit geringem Kraftaufwand zu bedienen ist. Außerdem ist es eine Aufgabe der vorliegenden Erfindung, die Entnahme von Teststreifen zu vereinfachen und für den Benutzer eindeutig kenntlich zu machen, wenn das Vorratsbehältnis nicht vollständig geschlossen ist. Obendrein soll das neuartige Vorratsbehältnis einfach herstellbar und kostengünstig sein.

Die vorgenannten Aufgaben wurden erfindungsgemäß gelöst durch ein Vorratsbehältnis für streifenförmige Testelemente enthaltend einen Köcher, der eine Öffnung besitzt, die durch einen Rand begrenzt ist. Testelemente, die sich im Köcher befinden, ein Verschlußteil, das über ein Scharnier mit dem Köcher verbunden ist und das an seiner Innenseite eine elastische Dichtung besitzt, die bei geschlossenem Vorratsbehältnis deformiert ist und dichtend an dem Rand des Köchers anliegt und die bei geöffnetem Vorratsbehältnis nicht deformiert ist, und eine lösbare Haltevorrichtung, die das Vorratsbehältnis in der geschlossenen Position hält, dadurch gekennzeichnet, daß das Verschlußteil aufgrund der mechanischen Spannung, unter der das Vorratsbehältnis durch die Deformation der Dichtung bei geschlossenem Vorratsbehältnis steht, bei gelöster Haltevorrichtung um die Achse des Scharniers verschwenkt ist und das Vorratsbehältnis dadurch sichtbar offensteht. Erfindungsgemäß ist es wichtig, daß die Öffnung des Vorratsbehältnisses nach Lösen der Haltevorrichtung von selbst, d. h. ohne ein weiteres Zutun des Benutzers, erfolgt. In der mechanischen Ruhelage ist das Vorratsbehältnis daher geöffnet.

Testelemente im Sinne der Erfindung besitzen eine Testzone, auf der eine Probenflüssigkeit, wie beispielsweise Blut oder Harn, aufgegeben wird. Die Probe erzeugt eine detektierbare Veränderung, wie beispielsweise eine Verfärbung, anhand derer die Anwesenheit und/oder Konzentration eines Analyten in der Probe ermittelt werden kann. Aufgrund der einfacheren Handhabbarkeit besitzen die Testelemente in der Regel eine langgestreckte Gestalt. Viele der im Handel befindlichen Testelemente sind gegen Feuchtigkeit empfindlich, so daß sie verschlossen aufbewahrt werden müssen, um die Verläßlichkeit des durchgeführten analytischen Tests zu gewährleisten.

Das erfindungsgemäße Vorratsbehältnis besitzt einen Köcher, in dem sich die Testelemente befinden. Der Köcher kann einen quadratischen, rechteckigen oder runden Querschnitt besitzen. Als vorteilhaft in der Handhabbarkeit hat sich ein ovaler Querschnitt des Köchers erwiesen. Der Köcher besitzt eine Öffnung, die durch einen Rand begrenzt ist. Es hat sich als vorteilhaft herausgestellt, wenn dieser Rand vollständig in einer Ebene liegt. Weiterhin ist es günstig, wenn die Öffnung nicht innerhalb einer Mantelfläche liegt, sondern eine gesamte Stirnseite des Köchers geöffnet ist, so daß die Randfläche der Köcheröffnung senkrecht zur Längsachse des Köchers ausgerichtet ist. Die Länge des Köchers richtet sich nach der Länge der streifenförmigen Testelemente. In der Praxis wird man die Länge des Köchers nicht wesentlich größer oder kleiner wählen als die Länge der Testelemente.

Als Material für den Köcher kommen Materialien mit einer ausreichenden Steifigkeit in Betracht. Insbesondere sind Kunststoffe wie Polyethylen, Polypropylen und Polystyrol geeignet.

Das Vorratsbehältnis besitzt weiterhin ein Verschlußteil, das über ein Scharnier mit dem Köcher verbunden ist. Die Form des Verschlußteiles ist so gewählt, daß sie wie eine Kappe die Öffnung des Köchers verschließen kann. Als Material für das Verschlußteil kommen insbesondere die für den Köcher genannten Materialien in Frage. Besonders bevorzugt ist es, wenn Köcher und Verschlußteil aus dem gleichen Material bestehen, da das Vorratsbehältnis dann einteilig hergestellt werden kann. Das Scharnier, das Verschlußteil und Köcher verbindet, kann beispielsweise ein Band- oder Gelenkscharnier sein. Vorteilhaft wird jedoch ein Filmscharnier verwendet, da in diesem Fall eine kostengünstige Herstellung im Spritzgußverfahren möglich ist. Ein wesentliches Merkmal der vorliegenden Erfindung ist es, daß an der Innenseite des Verschlußteiles eine elastische Dichtung angebracht ist, die so angeordnet ist, daß sie bei geschlossenem Vorratsbehältnis dichtend auf dem Rand des Köchers auf- oder an diesem Rand anliegt. Als Material für die Dichtung kommen Moosgummi, Styrolelastomere, Polyolefinelastomere, Polyamidelastomere, Polyesterelastomere und Polyurethanelastomere in Frage. Dichtringe aus den genannten Materialien können in die Innenseite des Verschlußteiles beispielsweise hineingeklebt oder hineingeschweißt sein. Bei einer besonders bevorzugten Ausführungsform der Erfindung wird der Verschlußteil in einem sogenannten Hart-/Weich-Spritzgießverfahren hergestellt. Bei diesem Verfahren wird zunächst der harte Verschlußteil gespritzt und das weiche Dichtungsgummi aufgespritzt. Umgekehrt ist auch ein Aufspritzen des Behälters auf das Dichtungsgummi möglich. Dieses Verfahren bietet auch den Vorteil einer höheren Prozeßsicherheit, da die Montage der Dichtung durch das Spritzgußverfahren vorgenommen wird und damit separate Herstellungsschritte und auch manuelle Fertigungsschritte vermieden werden können. Der Einsatz des Overmoulding-Verfahrens vermindert weiterhin die Herstellkosten.

Eine besonders geeignete Kombination ergibt sich bei diesem Verfahren, wenn für den Verschlußteil Polypropylen oder Polyamid eingesetzt wird und als elastisches Dichtmaterial Styrolelastomere oder Polyolefinelastomere gewählt werden. Erfindungsgemäß geeignete Elastomere besitzen eine Shore-Härte (DIN 53 505) von 30 bis 80, vorzugsweise zwischen 55 und 64.

Ein besonders wichtiger Aspekt ist die geometrische Anordnung der Dichtung relativ zum Rand der Köcheröffnung. Es hat sich als vorteilhaft herausgestellt, wenn die Dichtung in ihrem Querschnitt L-förmig ausgebildet ist und so angeordnet wird, daß zwischen Verschlußteil und Dichtung eine Nut entsteht, deren Abstand durch das kürzere Segment des L gegeben ist. Bei dieser Anordnung kommt der Rand des Köchers bei geschlossenem Vorratsbehältnis in dieser Nut zu liegen und die Dichtung drückt von der Innenseite des Köchers gegen den Köcherrand. Um eine ausreichende Dichtigkeit zu erzielen, wird die Breite der Nut zwischen Dichtung und Verschlußteil so gewählt, daß sie kleiner ist als die Dicke des Köchermaterials. Bei einem Verschließen wird daher der Köcherrand in diese Nut eingeklemmt, wobei die Dichtung deformiert wird. Aufgrund dieser Deformation steht das Vorratsbehältnis in geschlossenem Zustand unter einer mechanischen Spannung, die zu einer Öffnung des Vorratsbehältnisses führt, wenn die Haltevorrichtung entriegelt wird. Dieser Effekt kann durch eine Federspannung des Scharniers noch unterstützt werden. Es ist weiterhin möglich, zusätzlich zur beschriebenen Dichtung elastische Materialien im Bereich des Scharniers anzubringen, die bei verschlossenem Vorratsbehältnis komprimiert sind.

Während es bei Vorratsbehaltnissen des Standes der Technik (z. B. DE 43 28 815) beabsichtigt ist, die Ruheposition des Vorratsbehältnisses so zu wählen, daß das Behältnis geschlossen ist, wurde bei der vorliegenden Erfindung ein entgegengesetzter Weg eingeschlagen. Das Vorratsbehältnis steht sichtbar offen, wenn es nicht durch die Haltevorrichtung verriegelt ist. Dies bietet den Vorteil, daß ein unverschlossenes Vorratsbehältnis deutlich sichtbar offensteht. Damit kann verhindert werden, daß das Gefäß nicht vollständig verschlossen ist, ohne daß dies vom Benutzer erkannt werden kann. Vorzugsweise ist das Vorratsbehältnis in der Ruhelage um 20 bis 60 ° geöffnet.

Neben der bereits beschriebenen Ausführungsform, bei der sich der Rand des Köchers in einer Nut befindet, die auf einer Seite von der Dichtung begrenzt wird, ist auch eine Ausführungsform möglich, bei der die Dichtung auf den Rand des Köchers drückt. Diese Ausführungsform ist zwar scheinbar einfacher, stellt jedoch an die mechanische Abstimmung von Köcheröffnung und Dichtung höhere Anforderungen.

Wie bereits ausgeführt, befindet sich die mechanische Ruhelage des erfindungsgemäßen Vorratsbehältnisses in einer geöffneten Stellung. In dieser Position ist das Verschlußteil etwa um 30 ° gegenüber der geschlossenen Position verschwenkt. In der geschlossenen Position wird das Vorratsbehältnis durch eine Haltevorrichtung gehalten. Für diese Haltevorrichtung kommen die im Stand der Technik bekannten Ausführungsformen in Betracht. Insbesondere hat es sich als vorteilhaft erwiesen, wenn das Verschlußteil an seiner inneren Mantelfläche einen Zapfen oder eine Nase besitzt, die in geschlossener Stellung in eine Vertiefung in der äußeren Mantelfläche des Köchers einrastet. Durch Drücken auf eine der Vertiefung benachbarte Stelle des Köchers oder durch ein Ziehen an dem Verschlußteil kann die Haltevorrichtung manuell gelöst werden. Es ist weiterhin auch möglich, die Haltevorrichtung durch eine Deformation des Vorratsbehältnisses, beispielsweise ein Zusammendrücken, zu bewirken. Bei einer weiteren Ausführungsform besteht die Haltevorrichtung aus einer Vertiefung oder Ausnehmung in der Mantelfläche des Verschlußteiles, in die einen Zapfen, der sich auf der äußeren Mantelfläche des Köchers befindet, eingreift. Die Möglichkeiten zur Lösung der Haltevorrichtung entsprechen denen der vorgenannten Ausführungsform. Für beide exemplarisch genannten Haltevorrichtungen ist es vorteilhaft, wenn der Verschlußteil eine Lasche oder Zunge besitzt, die bei geschlossenem Vorratsbehältnis auf der Mantelfläche des Köchers zu liegen kommt. Die verschlußteilseitigen Elemente der Haltevorrichtung können vorteilhaft in den Bereich dieser Zunge verlegt werden.

Die Haltevorrichtung kann besonders handhabungsfreundlich durch einen Schieber geöffnet werden, der an dem Vorratsbehältnis angebracht ist. Der Schieber besitzt Führungselemente, die in entsprechende Führungselemente an dem Vorratsbehältnis so eingreifen, daß eine Verschiebung des Schiebers in Richtung der Längsachse des Vorratsbehältnisses möglich ist. Derartige Führungselemente können beispielsweise Nut- und Federkonstruktionen sein. Bei geschlossenem Vorratsbehältnis befindet sich der Schieber in einer ersten Position, in der sich eine Kante des Schiebers an dem Verschlußteil bzw. der Zunge befindet oder zumindest in der Nähe dieser Teile angeordnet ist. Durch Verschieben des Schiebers in Richtung auf das Verschlußteil wird die Haltevorrichtung entriegelt. Besonders vorteilhaft ist die Kante des Schiebers angeschrägt und der mit dieser Kante in Anlage kommende Bereich des Verschlußteils ebenfalls. Beim Verschieben des Schiebers drängt sich die Kante des Schiebers unter das Verschlußteil bzw. unter die Zunge und bewirkt so eine Entriegelung der Haltevorrichtung. Nach dem Verschieben des Schiebers befindet sich dieser in einer zweiten Position, aus der er durch manuelles Verschieben zurück in die erste Position bewegt werden kann. Bei Verwendung einer abgeschrägten Kante des Schiebers kann diese Zurückbewegung jedoch auch einfach durch Zudrücken des Verschlußteils erfolgen.

Die Verwendung einer Zunge an dem Verschlußteil bietet einen weiteren vorteilhaften Effekt. Zum Öffnen des Vorratsbehältnisses wird die Haltevorrichtung entriegelt, das Verschlußteil vom Benutzer um etwa 80 bis 150 ° um die Scharnierachse geschwenkt und die gesamte Vorrichtung etwas gekippt, so daß die im Köcher befindlichen Testelemente herausrutschen. Bei im Stand der Technik bekannten Vorrichtungen (US-4,717,018) muß der Anwender selbst dafür Sorge tragen, daß die Teststreifen nicht unkontrolliert herausfallen. Durch die vorstehend genannte Zunge an dem Verschlußteil kann jedoch sichergestellt werden, daß die Testelemente nicht unkontrolliert herausrutschen können. Wenn das Verschlußteil durch Verschwenken um ca. 80 bis 190 °, vorzugsweise 90 ° geöffnet wurde, befindet sich die Zunge des Verschlußteiles zur Öffnung des Köchers in einem Abstand, der im wesentlichen durch die Breite des Verschlußteiles vorgegeben ist. Vorteilhaft wird die Breite des Verschlußteiles so gewählt, daß sie kleiner ist als die Länge der Testelemente, jedoch groß genug, um eine einfache, manuelle Entnahme zu gewährleisten. Dies ist in praktischen Fällen dann der Fall, wenn die Breite des Verschlußteiles etwa im Bereich von 2 bis 4 cm liegt. Besitzt das Vorratsbehältnis die genannte Zunge an dem Verschlußteil, so rutschen die Testelemente bei Verkippen des Vorratsbehältnisses gegen die Zunge, von wo sie auf einfache Weise manuell entnommen werden können. Die Zunge übernimmt daher die Funktion einer Prallplatte. Weiterhin ist es günstig, wenn die Zunge an den Rändern gebogen ist, so daß sie sich in geschlossenem Zustand der Außenkontur des Köchers anpaßt. Die Krümmung der Zunge ist auch günstig für die Entnahme von Teststreifen, da diese durch die Krümmung an einem unbeabsichtigten seitlichen Herausrutschen gehindert werden.

Vorteilhaft kann das Vorratsbehältnis, durch ein Etikett, das über Köcher und Verschlußteil geklebt wird, versiegelt werden. Das Etikett dient somit als Originalitätsverschluß. Besonders vorteilhaft ist ein solcher Originalitätsverschluß einsetzbar, wenn das Verschlußteil eine Zunge besitzt, deren Außenseite mit dem Köcher eine durchgehende Kontur bildet.

Im Bereich der klinischen Diagnostik ist es häufig notwendig, entweder die Testelemente oder das Vorratsbehältnis für Testelemente dahingehend zu kennzeichnen, aus welcher Herstellungscharge die Testelemente stammen. Bei dem erfindungsgemäßen Vorratsbehältnis kann eine von der Außenseite zugängliche Kammer vorgesehen werden, die jedoch gegenüber dem Innenraum des Vorratsbehältnisses abgetrennt ist. In diese Kammer, die vorteilhaft schlitzförmig ausgebildet wird, kann ein Datenträger mit entsprechenden Informationen über die im Vorratsbehältnis enthaltenen Testträger aufbewahrt werden. Auf diese Weise ist es möglich, das Vorratsbehältnis nach Entleerung einer nochmaligen Verwendung zuzuführen, da beim Einfüllen neuer Testelemente lediglich der Datenträger ausgewechselt werden muß. Zur einwandfreien Bevorratung der Testelemente ist normalerweise auch ein Austausch des Trockenmittels notwendig.

Im Stand der Technik gebräuchliche Testelemente sind in der Regel empfindlich gegen Feuchtigkeit. Obwohl das erfindungsgemäße Vorratsbehältnis in verschlossenem Zustand einen guten Schutz gegen eindringende Feuchte bietet, ist es vorteilhaft, im Inneren des Vorratsbehältnisses ein Trockenmittel vorzusehen, da bei jedem Öffnen des Vorratsbehältnisses feuchte Umgebungsluft eindringt. Durch ein Trockenmittel im Inneren des Vorratsbehältnisses kann für die Testelemente eine längere einwandfreie Funktion gewährleistet werden. Vorteilhaft kann das Trockenmittel in den Boden des Köchers eingebracht werden, wo es durch eine feuchtigkeitsdurchlässige Membran (beispielsweise Karton) gegenüber den Teststreifen abgetrennt wird. Geeignete Trockenmittel sind im Stand der Technik hinlänglich bekannt. Geeignet sind beispielsweise Kieselgel, Molekularsiebe usw.

Zur Erfindung gehört weiterhin ein Verfahren zum Zurverfügungsstellen von Testelementen, bei dem ein mit Testelementen gefülltes Vorratsbehältnis durch Lösen der Haltevorrichtung geöffnet, darauf der Verschlußteil um die Scharnierachse verschwenkt und das Vorratsbehältnis so gekippt wird, daß die Testelemente gegen das Verschlußteil rutschen. Von hier können die Testelemente manuell entnommen werden. Nach der Entnahme wird das Vorratsbehältnis vom Benutzer durch Verschwenken des Verschlußteiles und Einrasten der Haltevorrichtung verschlossen.

Die vorliegende Erfindung wird anhand der folgenden Figuren näher erläutert:
- Fig. 1:: Außenansicht des Vorratsbehältnisses von der Seite
- Fig. 2:: Außenansicht des Vorratsbehältnisses von der Frontseite
- Fig. 3:: Querschnitt durch das Vorratsbehältnis entlang der Linie 3-3 in Fig. 2
- Fig. 4:: Querschnitt durch das Vorratsbehältnis mit zwei Öffnungsstellungen des Verschlußteiles
- Fig. 5:: Querschnitt einer zweiten Ausführungsform des Vorratsbehältnisses mit geöffnetem und geschlossenem Verschlußteil

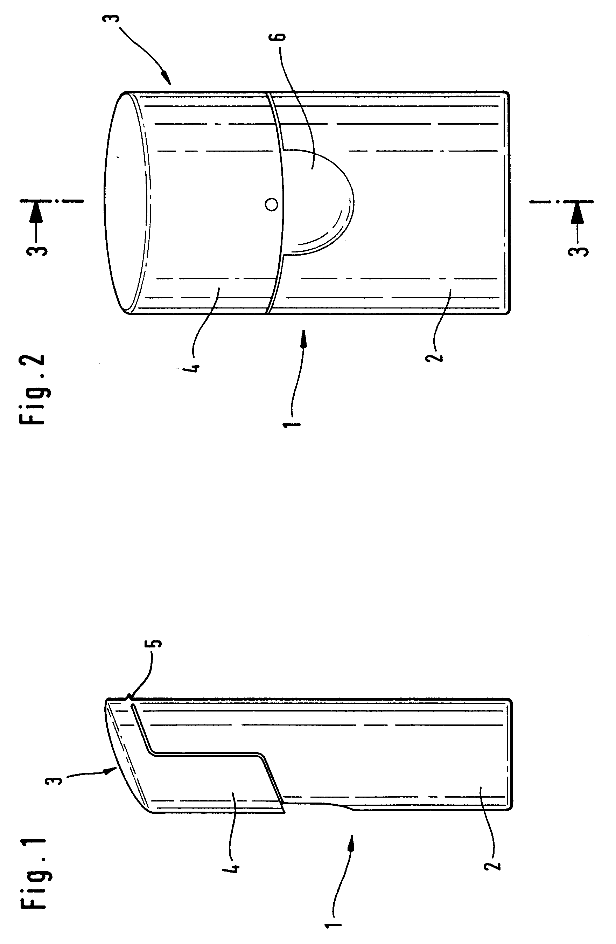

Fig. 1 zeigt eine Außenansicht eines erfindungsgemäßen Vorratsbehältnisses für streifenförmige Testelemente. Das Vorratsbehältnis (1) besitzt einen Köcher (2) und ein Verschlußteil (3), die über ein Filmscharnier (5) miteinander verbunden sind. Der Verschlußteil (3) besitzt eine Zunge (4), die bei geschlossenem Vorratsbehältnis an der äußeren Mantelfläche des Köchers (2) anliegt. Das in Fig. 1 dargestellte Vorratsbehältnis wurde einteilig im Spritzgußverfahren aus Polypropylen hergestellt.

Fig. 2 zeigt das Vorratsbehältnis (1) in Frontansicht. Es ist zu erkennen, daß der Querschnitt des Vorratsbehältnisses oval ist, was sich als günstig für die Handhabung erwiesen hat. In Fig. 2 ist weiterhin eine Vertiefung (6) im Köcher zu erkennen, die dazu dient, dem Benutzer eine Öffnung des Vorratsbehältnisses zu ermöglichen. Zum Öffnen kann der Benutzer entweder auf die Vertiefung (6) drücken und so die Haltevorrichtung entriegeln oder er kann dank der Vertiefung (6) unter den Rand der Zunge (4) drücken und so eine Entriegelung bewirken.

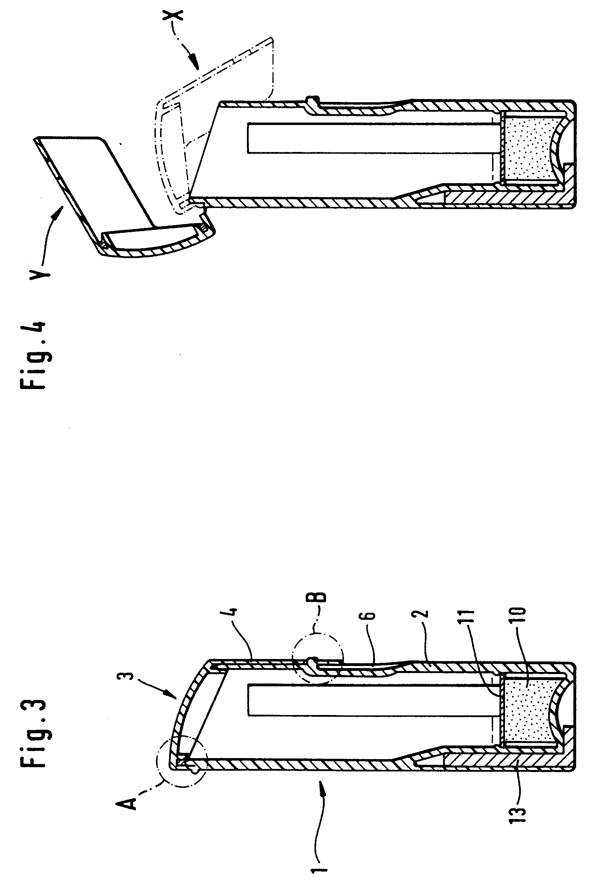

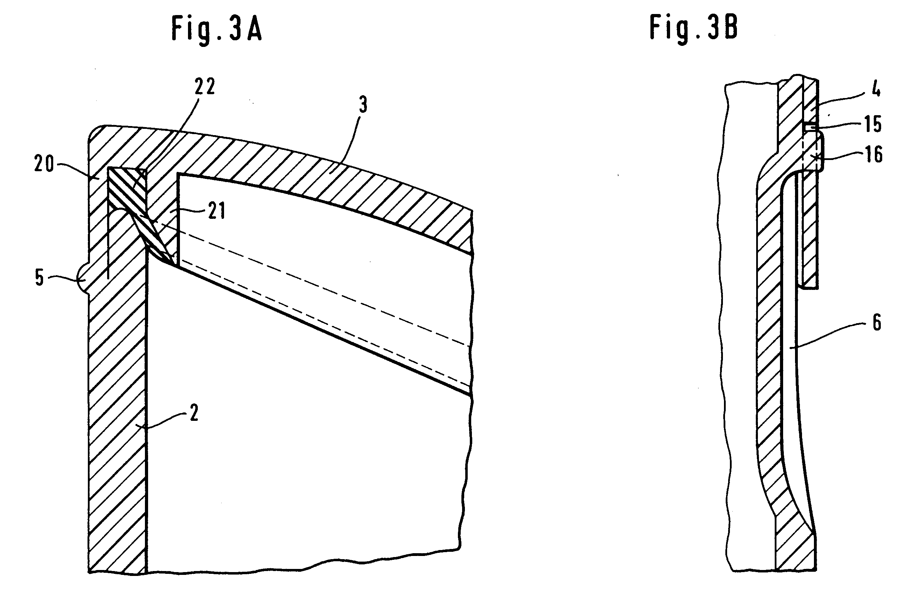

Fig. 3 zeigt einen Querschnitt durch ein erfindungsgemäßes Vorratsbehältnis entlang der in Fig. 2 eingezeichneten Linie 3-3. Fig. 3 zeigt, wie das Verschlußteil (3) und der Rand der Köcheröffnung ineinandergreifen. Detaillierter ist dies aus der Ausschnittsvergrößerung in Fig. 3A zu erkennen. Das Verschlußteil (3) besitzt eine äußere Kante (20), die eine Mantelfläche des Verschlußteiles bildet. Auf der Innenseite des Verschlußteiles (3) ist eine ringförmig umlaufende Kante (21) angebracht. Die Kanten (20) und (21) bilden eine umlaufende Nut. In dieser Nut befindet sich eine Dichtung (22) aus einem Styrolelastomeren. Bei geschlossenem Vorratsbehältnis drückt der Köcherrand die Dichtung (22) zusammen.

Aus Fig. 3 ist eine Haltevorrichtung zu erkennen. Die Ausschnittsvergrößerung in Fig. 3B zeigt eine weitere mögliche Ausführungsform der Haltevorrichtung. In der Zunge (4) des Verschlußteiles befindet sich eine Ausnehmung (15), die auf einem Zapfen (16) eingerastet ist. An den Zapfen (16) schließt sich die Vertiefung (6) im Köcher an, so daß bei Druck auf die Vertiefung (6) der Zapfen (16) von der Ausnehmung (15) wegbewegt und die Haltevorrichtung geöffnet wird. Aufgrund der mechanischen Spannung in der zusammengedrückten Dichtung (22) dreht sich das Verschlußteil (3) um die durch das Filmscharnier (5) vorgegebene Achse und das Verschlußteil (3) springt auf.

In Fig. 3 ist weiterhin eine Trockenmittelkammer (10) zu erkennen, die sich im Boden des Vorratsbehältnisses befindet. Die Trockenmittelkammer ist durch eine Abdeckplatte (11) aus Karton, die feuchtigkeitsdurchlässig ist, gegenüber dem restlichen Innenraum des Vorratsbehältnisses abgetrennt, so daß die Testelemente nicht direkt mit dem Trockenmittel in Kontakt treten können. Weiterhin ist in Fig. 3 eine schlitzförmige Kammer im Vorratsbehältnis zu erkennen, die von der Unterseite des Vorratsbehältnisses zugänglich ist. In dieser Kammer steckt ein Datenträger (13), der manuell aus der Kammer entnommen werden kann. Auf dem Datenträger können Informationen über die Testelemente, z. B. chargenspezifische Daten, in Form eines Strichcodes oder eines Chips aufgebracht sein.

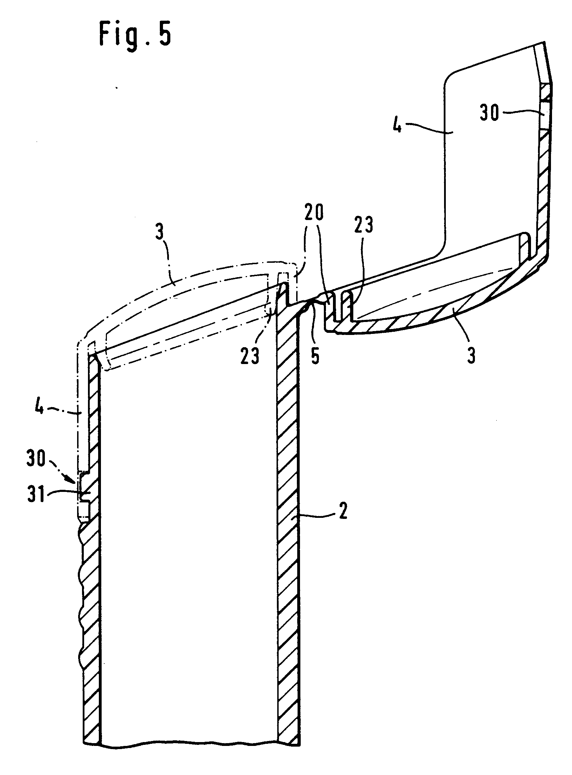

Fig. 4 zeigt zwei Öffnungsstellungen des Vorratsbehältnisses. Bei der Öffnungsstellung X ist das Verschlußteil etwa 30 ° gegenüber der vollständig geschlossenen Position geöffnet. In diese Position springt das Verschlußteil, wenn die Haltevorrichtung entriegelt wird.

Die Öffnungsstellung Y entspricht einer Stellung, in die das Verschlußteil vom Benutzer zur Entnahme von Testelementen gebracht wird. In dieser Stellung ist das Verschlußteil etwa 110 ° gegenüber der geschlossenen Position geöffnet.

Fig. 5 zeigt eine abgewandelte Ausführungsform des erfindungsgemäßen Vorratsbehältnisses. Diese Ausführungsform unterscheidet sich von der vorstehend beschriebenen bezüglich der Abdichtung gegenüber einem Eindringen von Feuchte.

Das Verschlußteil dieser Ausführungsform ist zweiteilig. Der erste Teil entspricht der ersten Ausführungsform und besitzt eine äußere Kante (20) sowie eine Zunge (4). Die Dichtung ist in diesem Fall durch eine umlaufende Ringwulst (23) mit einem L-förmigen Querschnitt gegeben. Die Ringwulst (23) ist so angebracht, daß sich zusammen mit der äußeren Kante (20) eine umlaufende Nut ergibt. Die Breite dieser Nut ist durch das kleinere Segment der L-förmigen Ringwulst gegeben. Das längere Teilsegment der Ringwulst (23) bildet einen inneren Rand, der analog der inneren Kante (21) der erstgenannten Ausführungsform ist. In Fig. 5 ist das Vorratsbehältnis sowohl in geöffneter Stellung als auch verschlossen dargestellt. Die geöffnete Stellung entspricht dem Zustand, in dem das Vorratsbehältnis aus der Spritzgußform entnommen wird. Beim Schließen wird das Filmscharnier (5) verbogen, hat jedoch aufgrund der Formgebung, in der es ursprünglich abgespritzt wurde, eine Tendenz, zumindest partiell in seine Ausgangslage zurückzukehren. Diese Federspannung des Filmscharniers unterstützt das Aufspringen des Vorratsbehältnisses, nachdem das Verschlußteil entriegelt wurde. In der Fig. 5 ist weiterhin zu erkennen, wie die Ringwulst (23) in der geschlossenen Stellung durch den Rand der Köcheröffnung deformiert wird. Der Rand der Köcheröffnung klemmt sich zwischen die äußere Kante (20) und die Ringwulst (23) und drückt dabei die Ringwulst nach innen. Wie bei der vorgenannten Ausführungsform bedingt die Deformation der elastischen Ringwulst eine mechanische Spannung, die eine Öffnung des Vorratsbehältnisses bewirkt, sobald die Haltevorrichtung entriegelt ist.

Die Haltevorrichtung der in Fig. 5 dargestellten Ausführungsform besteht aus einer Ausnehmung (30) in der Zunge (4) am Verschlußteil und einem Zapfen (31), der sich auf der äußeren Umfangsfläche des Köchers (2) befindet.

Anhand von Fig. 5 läßt sich ebenfalls der Einsatz von zusätzlichen Elastomeren im Bereich des Scharniers erklären. Hierzu werden Elastomere rechts und/oder links oberhalb des Scharniers (5) aufgebracht, die bei geschlossenem Vorratsbehältnis komprimiert werden. Als vorteilhaft hat es sich erwiesen, mehrere hochstehende Zapfen von Elastomeren aufzubringen.

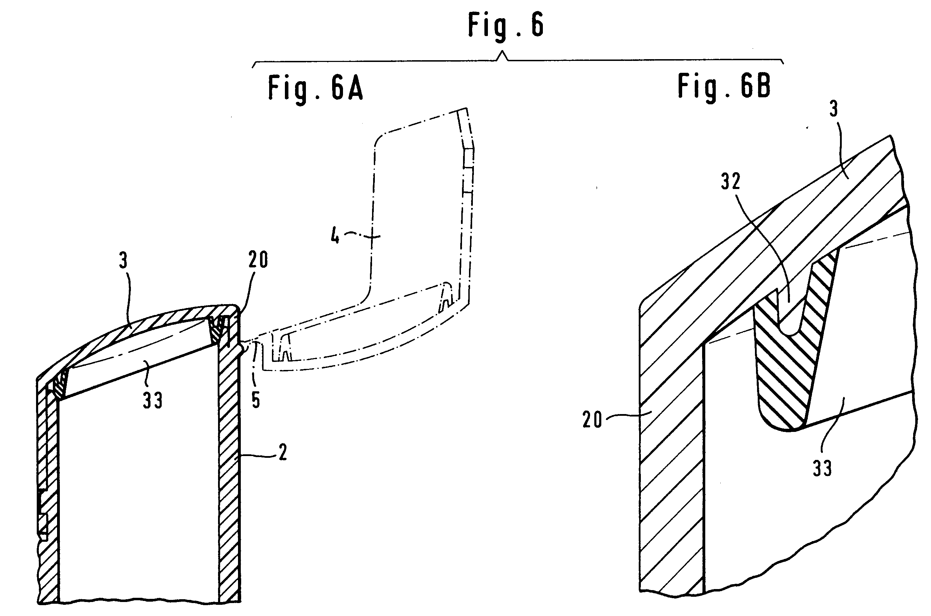

Eine weitere Ausführungsform des erfindungsgemäßen Vorratsbehältnisses zeigt Fig. 6. In Fig. 6A ist der obere Bereich des Vorratsbehältnisses zu erkennen. Generell entspricht diese Ausführungsform der in Fig. 5 dargestellten. Figuren 6A und 6B zeigen jedoch eine weitere Möglichkeit der Dichtung. An der Innenseite des Verschlußteiles (3) befindet sich eine ringförmig umlaufende Kante (32), die einteilig mit dem Verschlußteil (3) gefertigt wird. Um die ringförmig umlaufende Kante ist ein Dichtungsrand (33) gespritzt, der in seiner Form im wesentlichen der inneren Kante (21) des Verschlußteiles (Fig. 3A) entspricht. In Fig. 6A ist das Vorratsbehältnis geöffnet und geschlossen dargestellt. In der geschlossenen Position wird der Dichtungsrand (33) durch den Rand des Köchers deformiert und steht daher unter einer mechanischen Spannung. Nach dem Lösen der Haltevorrichtung springt das Vorratsbehältnis aufgrund dieser mechanischen Spannung auf.

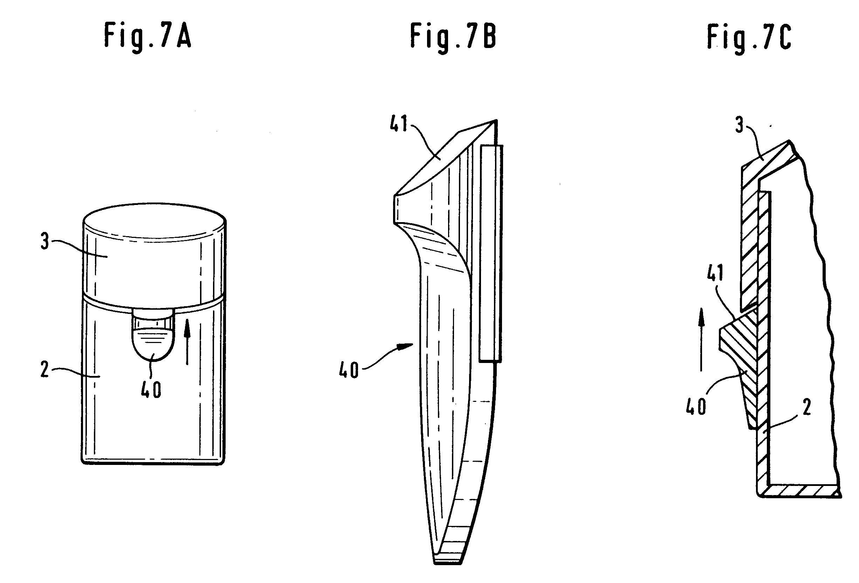

Fig. 7A zeigt eine Ausführungsform des Vorratsbehältnisses mit einem Schieber (40) zum Öffnen des Verschlußteils (3). Der Öffnungsvorgang erfolgt durch Bewegung des Schiebers in die mit dem Pfeil dargestellte Richtung.

Fig. 7B zeigt detallierter eine günstige Gestaltung des Schiebers (40) mit einer angeschrägten Kante (41). Das Zusammenwirken dieser Anschrägung mit dem Verschlußteil (3) ist deutlicher aus Fig. 7C zu erkennen. Der Schieber befindet sich partiell unterhalb des Verschlußteiles und drängt sich beim Verschieben wie ein Keil zwischen Verschlußteil und Köcher (2) des Vorratsbehältnisses.

### Bezugszeichenliste

- (1): Vorratsbehältnis
- (2): Köcher
- (3): Verschlußteil
- (4): Zunge an Verschlußteil
- (5): Filmscharnier
- (6): Vertiefung im Köcher
- (10): Trockenmittelkammer
- (11): Abdeckplatte für Trockenmittelkammer
- (13): Datenträger
- (15): Ausnehmung
- (16): Zapfen
- (20): Äußere Kante des Verschlußteils (3)
- (21): Innere Kante des Verschlußteils
- (22): Dichtung
- (23): Ringwulst (elastisch)
- (30): Ausnehmung
- (31): Zapfen
- (32): ringförmig umlaufende Kante
- (33): Dichtungsrand
- (40): Schieber
- (41): angeschrägte Kante

## Patentansprüche

1. Vorratsbehältnis (1) für streifenförmige Testelemente enthaltend
- einen Köcher (2), der eine Öffnung besitzt, die durch einen Rand begrenzt ist,
- Testelemente, die sich im Köcher (2) befinden,
- ein Verschlußteil (3), das über ein Scharnier (5) mit dem Köcher (2) verbunden ist und das an seiner Innenseite eine elastische Dichtung (22) besitzt, die bei geschlossenem Vorratsbehältnis (1) deformiert ist und dichtend an dem Rand des Köchers (2) anliegt und die bei geöffnetem Vorratsbehältnis (1) nicht deformiert ist, und
- eine lösbare Haltevorrichtung, die das Vorratsbehältnis (1) in der geschlossenen Position hält,
**dadurch gekennzeichnet, daß** das Verschlußteil (3) aufgrund der mechanischen Spannung, unter der das Vorratsbehältnis (1) durch die Deformation der Dichtung (22) bei geschlossenem Vorratsbehältnis (1) steht, bei gelöster Haltevorrichtung um die Achse des Scharniers (5) verschwenkt ist und das Vorratsbehältnis (1) dadurch sichtbar offensteht.

2. Vorratsbehältnis (1) gemäß Anspruch 1, bei dem der Verschlußteil (3) aus einem wenig elastischen Kunststoff und die Dichtung (22) an der Innenseite des Verschlußteiles (3) aus einem elastischen Kunststoff besteht, der durch ein Hart-/Weich-Spritzgußverfahren auf dem Verschlußteil (3) aufgebracht wurde.

3. Vorratsbehältnis (1) gemäß Anspruch 1 oder 2, bei dem die Dichtung (22) bei geschlossenem Vorratsbehältnis (1) komprimiert ist.

4. Vorratsbehältnis (1) gemäß Anspruch 1 oder 2, bei dem sich die Dichtung (22) zwischen zwei ringförmig umlaufenden Kanten (20, 21)befindet.

5. Vorratsbehältnis (1) gemäß Anspruch 1 oder 2, bei dem das Verschlußteil (3) an seiner Innenseite eine ringförmig umlaufende Kante (32) besitzt, auf die ein Dichtungsrand (33) aufgespritzt ist.

6. Vorratsbehältnis (1) gemäß Anspruch 1 oder 2, bei dem eine Dichtung (22) mit L-förmigem Querschnitt auf die Innenseite des Verschlußteiles (3) aufgespritzt ist.

7. Vorratsbehältnis (1) gemäß Anspruch 2, bei dem der wenig elastische Kunststoff des Verschlußteiles (3) Polypropylen oder Polyamid und der elastische Kunststoff der Dichtung (22) ein Styrol-Elastomer oder Polyolefin-Elastomer ist.

8. Vorratsbehältnis (1) gemäß Anspruch 1, mit einem ovalen Querschnitt.

9. Vorratsbehältnis (1) gemäß Anspruch 1, bei dem das Scharnier (5) ein Filmscharnier ist.

10. Vorratsbehältnis (1) gemäß Anspruch 1, bei dem Köcher (2), Scharnier (5) und Verschlußteil (3) einteilig in einem Spritzgußverfahren hergestellt sind.

11. Vorratsbehältnis (1) gemäß Anspruch 1, bei dem die lösbare Haltevorrichtung aus einer Ausnehmung (15) oder Vertiefung in der Mantelfläche des Verschlußteiles (3) und einem Zapfen (16) auf der äußeren Mantelfläche des Köchers (2) besteht und bei geschlossenem Vorratsbehältnis (1) der Zapfen (16) in die Ausnehmung (15) oder Vertiefung eingreift, so daß das Vorratsbehältnis (1) in einer verschlossenen Position gehalten wird.

12. Vorratsbehältnis (1) gemäß Anspruch 1, bei dem die lösbare Haltevorrichtung aus einer Nase an der inneren Mantelfläche des Verschlußteiles (3) und einer Vertiefung in der äußeren Mantelfläche des Köchers (2) besteht.

13. Vorratsbehältnis (1) gemäß Anspruch 11 oder 12, bei dem das Verschlußteil (3) an der gegenüberliegenden Seite zum Scharnier (5) eine Zunge (4) aufweist, und sich die Haltevorrichtung auf Höhe der Zunge (4) befindet.

14. Vorratsbehältnis (1) gemäß Anspruch 1, bei dem eine Öffnung des Verschlußteiles (3) durch Verschwenken um die Scharnierachse um 80-190° möglich ist, so daß bei Kippen des geöffneten Vorratsbehältnisses (1) die Testelemente aus dem Köcher (2) herausrutschen und durch die Zunge (4), die als Anschlag dient, vor einem Herausfallen aus dem Vorratsbehältnis (1) gehindert werden.

15. Vorratsbehältnis (1) gemäß Anspruch 1, das eine von der Außenseite zugängliche Kammer besitzt, die gegenüber dem Innenraum des Vorratsbehältnisses (1) abgetrennt ist und die zur Aufbewahrung eines Datenträgers (13) dient.

16. Vorratsbehältnis (1) gemäß Anspruch 1, bei dem sich an der Innenseite des Verschlußteiles (3) oder im Köcher (2) ein Trockenmittel befindet.

17. Vorratsbehältnis (1) gemäß Anspruch 1, das sich nach Lösen der Haltevorrichtung um 20 bis 60 ° öffnet.

18. Vorratsbehältnis (1) gemäß Anspruch 1, mit einem an dem Vorratsbehältnis (1) angebrachten Schieber (40), der eine Kante (41) besitzt, die in Richtung auf das Verschlußteil (3) bewegt werden kann und dadurch die Haltevorrichtung entriegelt.

19. Vorratsbehältnis (1) gemäß Anspruch 18, bei dem die Kante (41) angeschrägt ist.

20. Verfahren zum zur Verfügungstellen von Testelementen, bei dem ein mit Testelementen gefülltes Vorratsbehältnis (1) gemäß Anspruch 1 durch Lösen der Haltevorrichtung geöffnet, das Verschlußteil (3) um die Scharnierachse verschwenkt und das Vorratsbehältnis (1) so gekippt wird, daß die Testelemente gegen das Verschlußteil (3) rutschen.

## Claims

1. Storage container (1) for strip-shaped test elements comprising
- a holder (2) which has an opening bordered by a rim
- test elements which are located in the holder (2)
- a lid (3) which is connected to the holder (2) by a hinge (5) and has an elastic seal (22) on its inner side, which is deformed when the storage container (1) is closed and fits tightly against the rim of the holder (2) and is not deformed when the storage container (1) is opened and
- a releasable fastening device which holds the storage container (1) in the closed position
**wherein** the lid (3), due to the mechanical tension which acts on the storage container (1) when the seal (22) is deformed when the storage container (1) is closed, is rotated about the axis of the hinge (5) when the fastening device is released and as a result of which the storage container (1) is visibly open.

2. Storage container (1) as claimed in claim 1 in which the lid (3) is composed of a less elastic plastic and the seal (22) on the inside of the lid (3) is composed of an elastic plastic which is applied to the lid (3) by a hard-/soft-injection moulding process.

3. Storage container (1) as claimed in claim 1 or 2 in which the seal (22) is compressed when the storage container (1) is closed.

4. Storage container (1) as claimed in claim 1 or 2 in which the seal (22) is located between two ring-shaped peripheral edges (20, 21).

5. Storage container (1) as claimed in claim 1 or 2 in which the lid (3) has a ring-shaped peripheral edge (32) on its inner side onto which a sealing rim (33) is injected.

6. Storage container (1) as claimed in claim 1 or 2 in which a seal (22) having an L-shaped cross-section is injected onto the inner side of the lid (3).

7. Storage container (1) as claimed in claim 2 in which polypropylene or polyamide is the less elastic plastic of the lid (3) and a styrene elastomer or polyolefin elastomer is the elastic plastic of the seal (22).

8. Storage container (1) as claimed in claim 1 with an oval cross-section.

9. Storage container (1) as claimed in claim 1 in which the hinge (5) is a film hinge.

10. Storage container (1) as claimed in claim 1 in which the holder (2), hinge (5) and lid (3) are produced as a single component by means of an injection moulding process.

11. Storage container (1) as claimed in claim 1 in which the releasable fastening device consists of a recess (15) or indentation in the lateral surface of the lid (3) and a pin (16) on the outer lateral surface of the holder (2) and when the storage container (1) is closed the pin (16) engages in the recess or indentation such that the storage container (1) is held in a closed position.

12. Storage container (1) as claimed in claim 1 in which the releasable fastening device is composed of a projection on the inner lateral surface of the lid (3) and a recess in the outer lateral surface of the holder (2).

13. Storage container (1) as claimed in claim 11 or 12 in which the lid (3) has a tongue (4) on the opposite side to the hinge (5) and the fastening device is situated at the level of the tongue (4).

14. Storage container (1) as claimed in claim 1 in which the lid (3) can be opened by rotating the hinge about the axis by 80-190° so that the test elements slide out of the holder (2) when the opened storage container (1) is tipped and the tongue (4) acts as a stop preventing the test elements from falling out of the storage container (1).

15. Storage container (1) as claimed in claim 1 having a chamber accessible from the outside which is separated from the interior of the storage container (1) and which serves to house a data carrier.

16. Storage container (1) as claimed in claim 1 in which a desiccant is located on the inside of the lid (3) or in the holder (2).

17. Storage container (1) as claimed in claim 1 which opens by 20 to 60° after releasing the fastening device.

18. Storage container (1) as claimed in claim 1 having a slide (40) attached to the storage container (1) said slide having an edge (41) which can be moved towards the lid (3) and thereby releases the fastening device.

19. Storage container (1) as claimed in claim 18 in which the edge (41) is tapered.

20. Method for dispensing test elements wherein a storage container (1) as claimed in claim 1 filled with test elements is opened by releasing the fastening device, the lid (3) is rotated about the axis of the hinge and the storage container (1) is tipped such that the test elements slide against the lid (3).

## Revendications

1. Récipient de stockage (1) pour éléments de test en forme de bande renfermant :
- un étui (2), qui possède une ouverture, qui est limitée par un bord,
- des éléments de test, qui se trouvent dans l'étui (2).
- une partie de fermeture (3), qui est reliée à l'étui (2) par une charnière (5) et qui possède sur son côté interne un joint élastique (22), qui est déformé lorsque le récipient de stockage (1) est fermé et adhère uniformément au bord de l'étui (2) et qui n'est pas déformé lorsque le réservoir de stockage (1) est ouvert, et
- un dispositif de retenue détachable, qui retient le récipient de stockage (1) dans la position fermée,
caractérisé en ce que la partie de fermeture (3), en raison de la tension mécanique, sous laquelle se trouve le récipient de stockage (1) du fait de la déformation du joint (22) lorsque le récipient de stockage (1) est fermé, est pivotée autour de l'axe de la charnière (5) lorsque le dispositif de retenue est détaché et le récipient de stockage (1) est de ce fait ouvert de façon visible.

2. Récipient de stockage (1) selon la revendication 1, dans lequel la partie de fermeture (3) se compose d'une matière synthétique peu élastique et le joint (22) sur le côté interne de la partie de fermeture (3) se compose d'une matière synthétique élastique, qui a été appliquée sur la partie de fermeture (3) par un procédé de moulage par injection dur / mou.

3. Récipient de stockage (1) selon la revendication 1 ou 2, dans lequel le joint (22) est comprimé lorsque le récipient de stockage (1) est fermé.

4. Récipient de stockage (1) selon la revendication 1 ou 2, dans lequel le joint (22) se trouve entre deux arêtes périphériques (20, 21) annulaires.

5. Récipient de stockage (1) selon la revendication 1 ou 2, dans lequel la partie de fermeture (3) possède sur son côté interne une arête périphérique (32) annulaire, sur laquelle est moulé un bord d'étanchéité (33).

6. Récipient de stockage (1) selon la revendication 1 ou 2, dans lequel un joint (22) avec une section transversale en forme de L est moulé sur le côté interne de la partie de fermeture (3).

7. Récipient de stockage (1) selon la revendication 2, dans lequel la matière synthétique peu élastique de la partie de fermeture (3) est du polypropylène ou du polyamide et la matière synthétique élastique du joint (22) est un styrène-élastomère ou un polyoléfine-élastomère.

8. Récipient de stockage (1) selon la revendication 1, avec une section transversale ovale.

9. Récipient de stockage (1) selon la revendication 1, dans lequel la charnière (5) est une charnière à film.

10. Récipient de stockage (1) selon la revendication 1, dans lequel l'étui (2), la charnière (5) et la partie de fermeture (3) sont fabriqués d'une seule pièce dans un procédé de moulage par injection.

11. Récipient de stockage (1) selon la revendication 1, dans lequel le dispositif de retenue détachable se compose d'un évidement (15) ou creux dans la surface latérale de la partie de fermeture (3) et d'un tourillon (16) sur la surface latérale externe de l'étui (2) et lorsque le récipient de stockage (1) est fermé, le tourillon (16) engrène dans l'évidement (15) ou creux, de sorte que le récipient de stockage (1) est maintenu dans une position fermée.

12. Récipient de stockage (1) selon la revendication 1, dans lequel le dispositif de retenue détachable est composé d'un nez sur la surface latérale interne de la partie de fermeture (3) et d'un creux dans la surface latérale externe de l'étui (2).

13. Récipient de stockage (1) selon la revendication 11 ou 12, dans lequel la partie de fermeture (3) présente sur le côté opposé à la charnière (5) une langue (4), et le dispositif de retenue se trouve à la hauteur de la langue (4).

14. Récipient de stockage (1) selon la revendication 1, dans lequel une ouverture de la partie de fermeture (3) est possible par pivotement autour de l'axe de la charnière de 80 - 190°, si bien que lors du basculement du récipient de stockage ouvert (1), les éléments de test glissent à l'extérieur de l'étui (2) et grâce à la langue (4), qui sert de butée, ne peuvent pas tomber du récipient de stockage (1).

15. Récipient de stockage (1) selon la revendication 1, qui possède une chambre accessible par le côté externe, qui est séparée par rapport à l'espace interne du récipient de stockage (1) et qui sert à conserver un support de données (13).

16. Récipient de stockage (1) selon la revendication 1, dans lequel se trouve un dessiccateur sur le côté interne de la partie de fermeture (3) ou dans l'étui (2).

17. Récipient de stockage (1) selon la revendication 1, qui s'ouvre de 20 à 60° après détachement du dispositif de retenue.

18. Récipient de stockage (1) selon la revendication 1, avec une glissière (40) agencée sur le récipient de stockage (1), qui possède une arête (41) pouvant être déplacée dans le sens de la partie de fermeture (3) et, ce faisant, déverrouille le dispositif de retenue.

19. Récipient de stockage (1) selon la revendication 18, dans lequel l'arête (41) est biseautée.

20. Procédé pour la mise à disposition d'éléments de test, dans lequel un récipient de stockage (1) rempli d'éléments de test selon la revendication 1 est ouvert par détachement du dispositif de retenue, est pivoté autour de l'axe de la charnière et le récipient de stockage (1) est basculé de manière telle que les éléments de test glissent contre la partie de fermeture (3).
